# EUROPEAN PATENT SPECIFICATION

(11) **EP 0 652 021 B1**
(45) Date of publication and mention of the grant of the patent: **30.12.1998**
(21) Application number: 94117187.8
(22) Date of filing: 31.10.1994
(51) Int. Cl.: A61M 11/06

(54) **Micronized spray device**
Zerstäubungsgerät für mikronisierte Produkte
Pulvérisateur de produits micronisés

(30) Priority: 09.11.1993 IT MI932385
(43) Date of publication of application: 10.05.1995
(73) Proprietor: MEFAR S.p.A., 25073 Bovezzo (Brescia) (IT)
(72) Inventor: Ballini, Faustino, Bovezzo (Brescia) (IT); Merlin, Marco, Sirmione (Brescia) (IT)
(74) Representative: Modiano, Guido, Dr.-Ing.

(56) References cited:
- EP-A- 0 261 649
- BE-A- 473 165
- DE-C- 300 822

## Description

The present invention relates to a micronized spray device particularly for washing nasal and neighboring cavities, which in practice are the nasal fossae, the paranasal sinuses, the rhinopharynx, the pharynx, the auditory tube, and the middle ear.

Currently there are no devices for cleansing nasal fossae and neighboring fossae from mucopurulent secretions or scabs.

In particular, currently commercially available inhalation-therapy devices are unable to eliminate catarrhal secretions, since they produce a jet that is too finely atomized owing to their particular structure, which has obstacles, diaphragms, or bends between the pisper and the outlet: these characteristics allow to produce a very fine aerosol that is suitable to penetrate in the tracheobronchial region but loses the larger water particles that are instead useful for treating the upper respiratory tract.

EP-A-0 261 649 discloses a spray device of a similar structure as mentioned above, comprising a body with an opening which body forms an atomization chamber on the bottom whereof there is a region for containing a washing liquid in which a spray nozzle draws and said spray nozzle being associated with a compressed-air injector.

A principal aim of the present invention is instead to produce an abundant aerosol with large-diameter particles that can penetrate directly in the nasal fossae, in the rhinopharynx, and in the pharynx.

Inhalation-therapy devices are furthermore unable to obtain a direct effect with penetration of the water particles in the paranasal sinuses and in the middle ear, since they are not applied with positive pressure.

An object of the present invention is instead to produce and transmit a positive pressure from the atomizing chamber to the nasal fossae so that the patient can, by swallowing, cause the micelles thus produced to penetrate in the paranasal sinuses and in the middle ear.

Another object of the invention is indeed to eliminate the above described drawbacks by providing a micronized spray device for washing nasal and neighboring cavities, in which there is a micronized jet which is directed and introduced in the nostrils without any diaphragm, obstacle, or curve that can limit its flow-rate or vary the diameter of the micelles as produced by the pisper, and the pressure whereof can furthermore be discharged completely into the nasal fossae, producing a positive-pressure space during swallowing.

Within the scope of the above specified aim, a particular object of the invention is to provide a micronized spray device that can perform a deep cleaning action that can affect the paranasal sinuses and the middle ear without however applying pressures that might be harmful.

Another object of the present invention is to provide a micronized spray device that allows to collect in a delimited region the water that returns from the nasal fossae, preventing the contamination of the physiological washing water that is atomized.

Another object of the present invention is to provide a micronized spray device which, by virtue of its particular constructive characteristics, is capable of giving the greatest assurances of reliability and safety in use.

With this aim, these objects, and others in view which will become apparent hereinafter, there is provided a micronized spray device for washing nasal cavities, according to the invention, which is characterized in that it comprises a bell-shaped body which forms an atomization chamber on the bottom of which there is a region for containing a washing liquid in which a spray nozzle draws. The spray nozzle is associated with a compressed-air injector and is axially aligned with the inner outlet formed by said bell-shaped body. An outer skirt furthermore surrounds the bell-shaped body and forms an outer outlet at the inner outlet and a second chamber which is concentric to the first one to collect the catarrhal secretions that have been detached from the nasal cavities. The second chamber is connected to the outside to vent the pressure generated during expiration. The outer skirt is arrangeable in a first position, in which the outer outlet is spaced from the inner outlet to thereby form an interspace that is connected to the collection chamber, and in a second position, in which the outer outlet overlaps the inner outlet.

The particular characteristics and advantages of the invention will become apparent from the following detailed description of a preferred but not exclusive embodiment thereof, illustrated only by way of non-limitative example in the accompanying drawings, wherein:

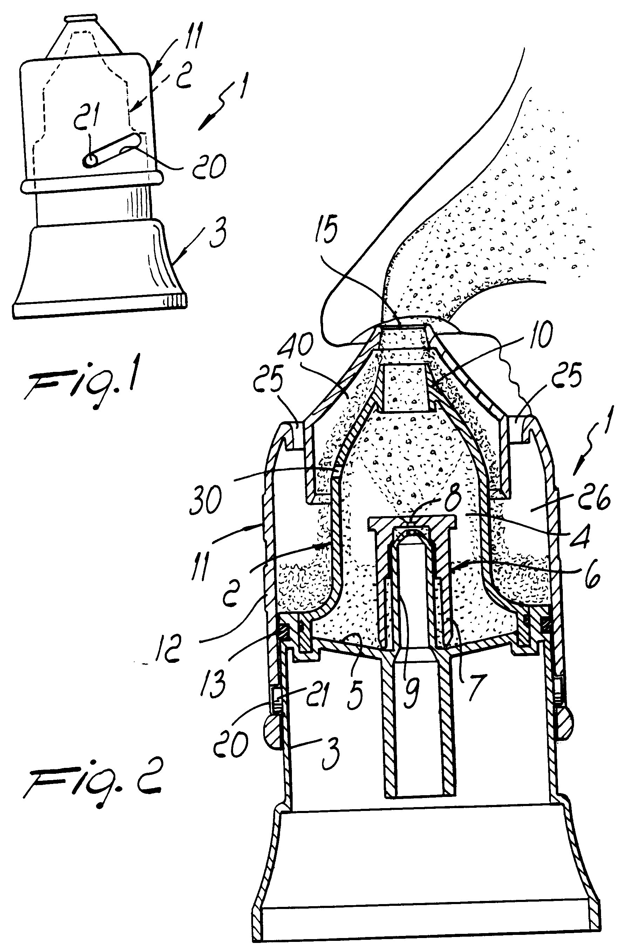

figure 1 is a schematic lateral elevation view of the device according to the invention;
figure 2 is an axial sectional view of the device with the outer skirt in the first position;
figure 3 is an axial sectional view of the device with the outer skirt in the second position.

With reference to the above figures, the micronized spray device for washing nasal cavities and neighboring cavities, according to the invention, is generally designated by the reference numeral 1 and comprises a bell-shaped body 2 which is detachably sealingly associable with a cap 3 to form an atomization chamber designated by the reference numeral 4.

At the bottom of the chamber 4 there is a region 5 that contains a washing liquid, which can be constituted by medicated or non-medicated physiological water or optionally by thermal water.

A spray nozzle, generally designated by the reference numeral 6, draws in the physiological water, has a cylindrical body 7 that is open towards the bottom, i.e. at the region 5, and has in an upward region a spraying hole 8 which is arranged at a compressed-air injector 9 which is located inside the cylindrical body 7 and forms in practice, together with the body 7, a region for aspirating the washing liquid which is injected by means of the spraying hole 8.

The particular structure that is adopted allows to create a jet of aqueous micelles whose diameter is greater than that of particles dispensed by conventional inhalation-therapy devices; a very large percentage of said micelles has a diameter of more than 10 microns, measured with a Malvern Size 2600 granulometer.

The jet of micronized aqueous particles is sent into the atomization chamber 4, which has an outlet 10 axially aligned with the spraying hole 8.

On the outside of the bell-shaped body 2 there is an outer skirt, designated by the reference numeral 11, having a lateral body 12 that is sealingly coupled externally to the bell-shaped body by virtue of the presence of an O-ring 13.

The skirt 11 forms, in an upward region, an outer outlet 15 which is arranged correspondingly and is axially aligned with the inner outlet 10.

Means are also provided for movably connecting said outer skirt 11, in an axial direction, with respect to the bell-shaped body; said means are advantageously constituted by at least one helical slot 20 which is formed on the outer skirt and in which there engages a pin 21 that protrudes from the cap 3 which is rigidly coupled to the bell-shaped body 2.

Furthermore, the outer skirt has, towards its upper part, ports 25 that connect to the outside the chamber 26 that collects the return water, which is constituted by the washing liquid and by the catarrhal material that has been detached from the nasal cavities.

There is also a pressure control valve element for discharging the excess pressure from said nebulization chamber 4; said valve element is constituted by a hole 30 which is formed in the upper part of the bell-shaped body 2 and is connected to the chamber 26.

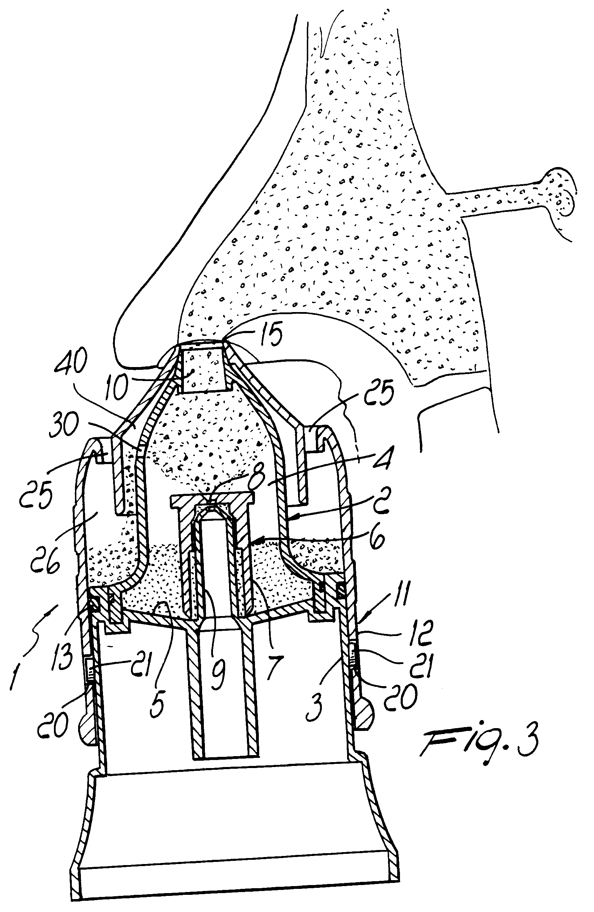

The outer skirt 11 can be arranged in a first position, shown in figure 2, in which the outer outlet 15 is spaced from the inner outlet 10, in practice forming an interspace 40 that allows the physiological material that has been detached from the nasal cavities to pass directly inside the chamber 26, preventing said physiological material from mixing with the washing liquid provided in the region 5. The outer skirt 11 can furthermore be arranged as shown in figure 3, in a second position, in which the inner outlet 10 enters the outer outlet to allow to penetrate in the paranasal cavities and in the middle ear and achieve the effect of cleansing and eliminating the catarrh and/or conveying the medicated constituents contained in addition to the washing water or thermal water.

In practical operation, during a first step the outer skirt is placed in the first position; when the compressed air is introduced by means of the injector, a jet of micelles of physiological water is obtained with particles larger than 10 microns in diameter which is atomized directly in the nostrils under a certain degree of pressure.

The abundant hydration of the nasal and neighboring cavities obtained with the micronized jet dissolves mucus and catarrh and detaches them due to the pressure applied to the mucous membranes.

The removed catarrhal and scab material, dissolved in the physiological water, is deposited by gravity in the collection chamber 26 without mixing with the stream that leaves the atomization chamber 4 by virtue of the pressure that escapes from the chamber, which does not allow the mucus to fall back inside it.

The pressure generated by the respiration of the patient during expiration is mainly discharged with the contralateral nostril and partly through the interspace 40 and the ports 25 that provide a connection to the outside.

After the first above described step of washing the nasal fossae, the rhinopharynx, and the pharynx, it may be necessary to perform a second washing step to clean the paranasal sinuses and open the auditory tube, restoring their functionality and thus normalizing the pressure of the middle ear with respect to the outside.

During this second step, the outer skirt of the device is placed in the second position, so as to fully close the interspace 40.

In order to clean the paranasal sinuses and open the auditory tube, the patient must swallow as frequently as possible, for example by sucking candy.

During these swallowing actions, a positive pressure is produced inside the nasal fossae and is caused by the pressure of the atomization obtained in the chamber 4.

This positive pressure is discharged towards all the neighboring cavities, thus into the paranasal cavities, into the auditory tube, and into the middle ear.

This also causes the aqueous micelles to move towards said cavities, cleansing them by diluting the mucus, by equalizing and normalizing the internal pressure of these cavities with respect to the external pressure, and by producing the pharmacological effect of any medical or thermal substances contained in the washing liquid.

During this step it is absolutely necessary to avoid creating dangerous pressure values, since an excessive pressure might cause internal lesions.

The compressed air that generates the micronized jet is normally set to 170 +- 10 kPa. During atomization, with the outer skirt arranged so as to fully close the interspace 40, during the swallowing actions of the patient the internal pressure increases in the atomization chamber but can partly vent by means of the safety hole 30 and partly open the auditory tubes and cause the atomised liquid to move towards the paranasal sinuses.

Furthermore, the liquid arriving from the nasal cavity and deposited in the chamber 26 is discharged through the hole 30.

The overpressure vented by virtue of the valve means constituted by the hole 30 discharges freely into the atmosphere by means of the ports 25.

From the above description it is thus evident that the invention achieves the intended aim and objects, and in particular the fact is stressed that a device is provided which, by sending into the nasal cavities an atomized jet in which a high percentage of its aqueous micelles are over 10 microns in size, allows to perform deep washing with optimum pressure values which in any case never reach dangerous levels.

The catarrhal material that is detached from the nasal cavities is furthermore not mixed with the washing liquid, which thus always has the best characteristics.

The invention thus conceived is susceptible of numerous modifications and variations, all of which are however within the scope of the appended claims.

In practice, the materials employed, so long as they are compatible with the specific use, as well as the contingent shapes and dimensions, may be any according to the requirements.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. Micronized spray device (1) for washing nasal and neighboring cavities, comprising a bell-shaped body (2) which forms an atomization chamber (4) on the bottom whereof there is a region (5) for containing a washing liquid in which a spray nozzle (6) draws, said spray nozzle being associated with a compressed-air injector (9) and being axially aligned with the inner outlet (10) formed by said bell-shaped body, an outer skirt (11) furthermore surrounding said bell-shaped body and forming an outer outlet (15) at said inner outlet and a chamber (26) for collecting the catarrhal material detached from the nasal cavities which is connected to the outside to vent the pressure generated during expiration, said outer skirt being arrangeable in a first position, in which said outer outlet is spaced from said inner outlet, forming an interspace (40) that is connected to said collection chamber, and in a second position, in which said outer outlet overlaps said inner outlet.

2. Device according to claim 1, wherein said bell-shaped body is sealingly detachably associable, in a downward region, with a cap (3) to form said atomization chamber.

3. Device according to one or more of the preceding claims, characterized in that said spray nozzle (6) comprises a cylindrical body (7) that is open towards the bottom and has, in an upward region, a spraying hole (8) which is located at said pressurized air injector.

4. Device according to one or more of the preceding claims, characterized in that it produces in output a jet of aqueous micelles in which a large percentage has a size above 10 microns.

5. Device according to one or more of the preceding claims, characterized in that said outer skirt (11) comprises a lateral body (12) that can be sealingly coupled on the outside of said bell-shaped body (2).

6. Device according to one or more of the preceding claims, characterized in that it comprises means (20,21) for moving said outer skirt with respect to said bell-shaped body to pass between said first position and said second position.

7. Device according to claim 6, characterized in that said movement means comprise at least one helical slot (20) which is formed on said outer skirt and in which at least one pin (21) engages, said pin protruding from the cap (3) that is rigidly coupled to said bell-shaped body (2).

8. Device according to one or more of the preceding claims, characterized in that it comprises a pressure control valve element (30) for discharging the excess pressure in said atomization chamber (4).

9. Device according to claim 8, characterized in that said control valve element is constituted by a through hole (30) formed in said bell-shaped body (2) and suitable to connect said atomization chamber (4) with said, collection chamber (26).

## Patentansprüche

1. Zerstäubungsgerät (1) für mikronisierte Produkte zum Waschen der Nasen- und benachbarter Hohlräume, umfassend einen glockenförmigen Körper (2), welcher eine Atomisierkammer (4) bildet, an deren Boden ein Bereich (5) zum Enthalten einer Waschflüssigkeit vorhanden ist, in welchen sich eine Spraydüse (6) erstreckt, dieser Spraydüse ein Druckluftinjektor (9) zugeordnet ist und diese axial fluchtet mit einem durch den glockenförmigen Körper gebildeten inneren Auslaß (10), weiterhin eine äußere Einfassung (11), die den glockenförmigen Körper umgibt und am inneren Auslaß einen äußeren Auslaß (15) und eine Kammer (26) zum Sammeln des von den Nasenhohlräumen gelösten katarrhalischen Materials bildet, welche mit der Außenseite verbunden ist, um den während des Ausatmens erzeugten Druck zu entlüften, wobei diese äußere Einfassung anordenbar ist in eine erste Stellung, bei welcher der äußere Auslaß im Abstand zum inneren Auslaß sich befindet und einen Zwischenraum (40) bildet, der mit der Sammelkammer verbunden ist, und in eine zweite Stellung, in welcher der äußere Auslaß den inneren Auslaß überlappt.

2. Gerät nach Anspruch 1, bei welchem der glockenförmige Körper in einem unteren Bereich zur Bildung der Atomisierkammer abdichtend lösbar verbindbar mit einer Kappe (3) ist.

3. Gerät nach einem oder mehreren der vorhergehenden Ansprüche, **dadurch gekennzeichnet**, daß die Spraydüse (6) einen zylindrischen Körper (7) umfaßt, der zum Boden hin offen ist und in einem oberen Bereich ein Sprayöffnung (8) aufweist, welches bei dem Druckluftinjektor angeordnet ist.

4. Gerät nach einem oder mehreren der vorhergehenden Ansprüche, **dadurch gekennzeichnet**, daß es im Auslaß einen Strahl von wässrigen Micellen erzeugt, in welchem ein großer Prozentteil eine Größe über 10 Mikron aufweist.

5. Gerät nach einem oder mehreren der vorhergehenden Ansprüche, **dadurch gekennzeichnet**, daß die äußere Einfassung (11) einen seitlichen Körper (12) umfaßt, welcher mit der Außenseite des glockenförmigen Körpers (2) abdichtend verbunden werden kann.

6. Gerät nach einem oder mehreren der vorhergehenden Ansprüche, **dadurch gekennzeichnet**, daß es Mittel (20, 21) zum Bewegen der äußeren Einfassung in Bezug auf den glockenförmigen Körper zum Übergang zwischen der ersten Stellung und der zweiten Stellung umfaßt.

7. Gerät nach Anspruch 6, **dadurch gekennzeichnet**, daß die Bewegungsmittel mindestens einen schraubenförmigen Schlitz (20) umfassen, welcher an der äußeren Einfassung angeformt ist und in welchen mindestens ein Stift (21) eingreift, wobei dieser Stift von der Kappe (3) absteht, welche starr mit dem glockenförmigen Körper (2) verbunden ist.

8. Gerät nach einem oder mehreren der vorhergehenden Ansprüche, **dadurch gekennzeichnet**, daß es zum Entlasten des Überdrucks in der Atomisierkammer (4) ein Drucksteuerventilelement (30) umfaßt.

9. Gerät nach Anspruch 8, **dadurch gekennzeichnet**, daß das Steuerventilelement gebildet wird durch ein im glockenförmigen Körper (2) gebildetes und zum Verbinden der Atomisierkammer (4) mit der Sammelkammer (26) geeignetes Durchlaßloch.

## Revendications

1. Pulvérisateur (1) de produits micronisés pour nettoyer les cavités nasales et avoisinantes, comprenant un corps en forme de cloche (2) qui forme une chambre d'atomisation (4) au fond de laquelle se trouve une zone (5) pour contenir un liquide de nettoyage où aspire une buse de pulvérisation (6), ladite buse de pulvérisation étant associée à un injecteur d'air comprimé (9) et étant axialement alignée avec la sortie interne (10) formée par ledit corps en forme de cloche, une jupe externe (11) entourant par ailleurs ledit corps en forme de cloche et formant une sortie externe (15) au niveau de ladite sortie interne et une chambre (26) pour recueillir la matière catarrhale détachée des cavités nasales, qui est reliée à l'extérieur pour évacuer la pression engendrée pendant l'expiration, ladite jupe externe pouvant être agencée dans une première position, dans laquelle ladite sortie externe est espacée de ladite sortie interne, formant une espacement (40) qui est relié à ladite chambre de collecte, et dans une seconde position, dans laquelle ladite sortie externe chevauche ladite sortie interne.

2. Dispositif selon la revendication 1, dans lequel ledit corps en forme de cloche peut-être associé, de façon amovible et étanche, dans une zone inférieure, à un capuchon (3) pour former ladite chambre d'atomisation.

3. Dispositif selon une ou plusieurs des revendications précédentes,
caractérisé en ce que ladite buse de pulvérisation (6) comprend un corps cylindrique (7) qui est ouvert vers le fond et présente, dans une zone supérieure, un trou de pulvérisation (8) qui est situé au niveau dudit injecteur d'air sous pression.

4. Dispositif selon une ou plusieurs des revendications précédentes,
caractérisé en ce qu'il produit, en sortie, un jet de micelles aqueuses, dans lesquelles un pourcentage important présente une taille au-dessus de 10 micromètres.

5. Dispositif selon une ou plusieurs des revendications précédentes,
caractérisé en ce que ladite jupe externe (11) comprend un corps latéral (12) qui peut être couplé de façon étanche sur l'extérieur dudit corps en forme de cloche (2).

6. Dispositif selon une ou plusieurs des revendications précédentes,
caractérisé en ce qu'il comprend des moyens (20,21) pour déplacer ladite jupe externe par rapport audit corps en forme de cloche pour passer entre ladite première position et ladite seconde position.

7. Dispositif selon la revendication 6,
caractérisé en ce que lesdits moyens de déplacement comprennent au moins une fente hélicoïdale (20) qui est formée sur ladite jupe externe et dans laquelle s'engage au moins une broche (21), ladite broche faisant saillie du capuchon (3) qui est coupé rigidement audit corps en forme de cloche (2).

8. Dispositif selon une ou plusieurs des revendications précédentes,
caractérisé en ce qu'il comprend un élément de vanne de commande de pression (30) pour évacuer la pression en excès dans ladite chambre d'atomisation (4).

9. Dispositif selon la revendication 8,
caractérisé en ce que ledit élément de vanne de commande est constitué par un trou traversant (30) formé dans ledit corps en forme de cloche (2) et approprié pour relier ladite chambre d'atomisation (4) à ladite chambre de collecte (26).
